# EUROPÄISCHE PATENTANMELDUNG

(11) **EP 3 273 235 A2**
(43) Veröffentlichungstag der Anmeldung: **24.01.2018**
(21) Anmeldenummer: 17001065.6
(22) Anmeldetag: 23.06.2017
(51) Int. Cl.: G01N 33/03

(54) **ÖLMESSGERÄT UND VERFAHREN ZUR ÜBERWACHUNG EINES IN EINEM BECKEN BEFINDLICHEN ÖLS**

(30) Priorität: 24.06.2016 DE 102016007668
(71) Anmelder: Testo SE & Co. KGaA, 79853 Lenzkirch (DE)
(72) Erfinder: Götz, Meinrad, 79848 Bonndorf (DE); Münzer, Markus, 78183 Hüfingen (DE)
(74) Vertreter: Börjes-Pestalozza, Henrich

(57) **Zusammenfassung**

Zur Überwachung eines in einem Becken 19 befindlichen Öls wird vorgeschlagen, ein Ölmessgerät 1 mit einer starren oder flexiblen Sonde 2 auszustatten, sodass mit Hilfe einer in dem Ölmessgerät 1 integrierten Pumpe 5 Öl aus dem Becken 19 über eine Einlassöffnung 3 am proximalen Ende der Sonde 2 in eine an die Sonde angeschlossene Messkammer 4 des Ölmessgeräts 1 förderbar ist. Dabei weist das Ölmessgerät 1 ein Messmittel zur Bestimmung der Qualität von in der Messkammer 4 befindlichem Öl auf und die Pumpe 5, in Verbindung mit der Sonde 2, ist so eingerichtet, dass Öl von der Messkammer 4 an die Einlassöffnung 3 und damit an das Becken 19 rückführbar ist (vgl. Fig. 2).

## Beschreibung

Die Erfindung betrifft ein Ölmessgerät zur Bestimmung und/oder Überwachung der Temperatur und/oder der Qualität eines in einem Becken befindlichen Öls.

Die Erfindung betrifft weiter ein Verfahren zur Überwachung eines in einem Becken befindlichen Öls. Hierbei können je nach Anwendung verschiedene Parameter für die Überwachung von Interesse sein, wie beispielsweise die Temperatur und Qualität des Öls oder etwa die Anzahl und/oder Dauer von Erhitzungszyklen, welches das Öl im Gebrauch durchläuft.

Im Stand der Technik lassen sich beispielsweise zahlreiche Haushalts- und/oder Gastronomiegeräte auffinden, die Becken, insbesondere elektrisch beheizbare Becken, aufweisen, welche im Betrieb mit flüssigem Öl oder Fett befüllt sind. Gerade in Großküchen kommen beispielsweise ortsfeste Fritteusen zum Einsatz, bei denen eine, vorzugsweise kontinuierliche, Überwachung der Temperatur des flüssigen Öls oder Fetts sowie, in regelmäßigen Abständen, eine Messung der Qualität des Öls oder Fetts aus Gründen der Lebensmittelhygiene erforderlich ist.

In Bezug auf Fritteusen bieten gängige Geräte beispielsweise die Möglichkeit, die Qualität des Frittieröls im Betrieb zu überwachen. Dazu wird typischerweise zwischen einem Frittierbecken und einem Filterbecken eine Rückleitung vorgesehen, wobei entweder in der Rückleitung oder abgezweigt von der Rückleitung ein Sensor vorgesehen ist, um die Qualität des zurückgepumpten Öls zu überprüfen.

Insbesondere für Fritteusen sind somit aus dem Stand der Technik Messsysteme bekannt mit Sensoren, die in der Wand eines das Öl aufnehmenden Beckens oder in einem daran angeschlossenen Filterkreislauf des Öls ortsfest verbaut sind.

Derartige Messsysteme können nicht ohne größere bauliche Änderungen dem Gerät entnommen werden. Somit bieten solche nicht-modularen Systeme insbesondere keine Möglichkeit, eine wie eingangs beschriebene Messfunktionalität an bereits bestehenden Fritteusen ohne fest verbaute Sensoren bei geringem Aufwand nachzurüsten. Mit bestehenden Systemen ist eine solche Nachrüstung aufwendig und teuer und es ist bei diesen auch nicht vorgesehen, mehrere Becken gleichzeitig oder flexibel zu überwachen.

Gerade für eine solche Nachrüstbarkeit und flexible Überwachung besteht jedoch ein erhöhter Bedarf, da einerseits die Anforderungen an die Überwachung der Lebensmittelhygiene stetig steigen und andererseits hohe Kosten für eine Nachrüstung beziehungsweise Neuanschaffungen von Geräten aus wirtschaftlichen Gründen zu vermeiden sind.

Typische Prozesse welche die Qualität des Öls, oder genauer die Qualität der Mischung aus flüssigem Öl oder Fett und darin befindlichen anderen Substanzen, beeinflussen sind der Zerfall von Öl-Molekülen sowie die Aufnahme von Wasser. Beide Prozesse führen in der Regel zu einer Änderung der elektrischen Eigenschaften des Öls, insbesondere zu einem Anstieg der elektrischen Permittivität ε des Öls, die auch als dielektrische Leitfähigkeit bezeichnet wird.

Es ist bekannt, dass durch eine Messung der Permittivität ε des Öls auf die Qualität des Öls, das heißt insbesondere auf die Reinheit des Öls und auf den Wasseranteil im Öl, zurückgeschlossen werden kann.

Um verlässliche Aussagen über die Qualität des Öls anhand einer Messung der Permittivität ε treffen zu können ist jedoch eine parallele Temperaturmessung erforderlich, da Wasser bei Temperaturanstieg aus dem Öl verdampft, und somit erst nach dem Verdampfen eine verlässliche Aussage möglich ist.

Der Erfindung liegt die Aufgabe zugrunde, ein flexibel handhabbares Ölmessgerät bereit zu stellen, mit dem die Qualität eines in einem Becken befindlichen Öls sowie vorzugsweise auch seine Temperatur einfach überwachbar ist.

Zur Lösung dieser Aufgabe sind erfindungsgemäß die Merkmale des Anspruchs 1 vorgesehen. Insbesondere wird somit erfindungsgemäß zur Lösung der Aufgabe bei einem Ölmessgerät der eingangs beschriebenen Art vorgeschlagen, dass das Ölmessgerät eine Sonde umfasst, die an ihrem distalen Ende eine Einlassöffnung aufweist, wobei an einem proximalen Ende der Sonde eine mit Öl befüllbare und an die Sonde angeschlossene Messkammer und eine Pumpe zur Förderung von Öl von der Einlassöffnung zur Messkammer angeordnet sind, wobei das Ölmessgerät ein Messmittel zur Bestimmung der Qualität des Öls aufweist und wobei die Pumpe zur Rückführung von in der Messkammer untersuchtem Öl zur Einlassöffnung eingerichtet ist.

Bei dieser erfindungsgemäßen Ausgestaltung des Ölmessgeräts ist von Vorteil, dass das Ölmessgerät alle Mittel zur Verfügung stellt, um das zu untersuchende Öl aus dem Becken zu entnehmen, zu untersuchen und anschließend wieder an das Becken zurückzugeben. Aufgrund der Eigenständigkeit des Ölmessgeräts sind somit größere bauliche Veränderungen an dem Becken verzichtbar. Somit kann das Ölmessgerät flexibel auch an mehreren Becken eingesetzt werden, wodurch die Gebrauchseigenschaften des Ölmessgeräts wesentlich verbessert werden. Insbesondere kann das Ölmessgerät somit modular an bestehenden Geräten mit Ölbädern, insbesondere Haushalts- oder Gastronomiegeräten mit beheizten Ölbädern, ohne größere bauliche Veränderungen derselben, nachgerüstet werden. Dadurch wird, insbesondere für bereits vorhandene Geräte, eine sichere und preisgünstige Möglichkeit eröffnet, das Öl während des Betriebs zu überwachen und zugehörige Messdaten zu erfassen und aufzuzeichnen.

Erfindungsgemäß kann das Messmittel zur Bestimmung der Ölqualität vorzugsweise in oder an der Messkammer angeordnet sein. Dadurch kann eine kompakte Bauweise des Ölmessgeräts sowie eine hohe Messgenauigkeit erreicht werden.

Eine weitere vorteilhafte Ausführung kann darin bestehen, dass die Pumpe zur Rückführung von untersuchtem Öl über die Sonde an das Becken eingerichtet ist. Damit ist vermeidbar, dass der Ölstand im Becken durch wiederholte Entnahmen von Öl für Messungen absinkt und somit Öl nachgefüllt werden muss. Zudem besteht bei dieser Ausführungsform keine Notwendigkeit, bereits untersuchtes Öl zu entsorgen.

Um das Ölmessgerät beispielsweise an bestehenden Fritteusen ohne bauliche Abänderungen derselben anordnen beziehungsweise in diese Geräte integrieren zu können ist es erfindungsgemäß modular aufgebaut: das Ölmessgerät stellt ein eigenständiges Modul dar, welches voll funktionsfähig ist, sobald eine Energieversorgung bereit gestellt wird. Diese Modularität des Systems erlaubt einen problemlosen Transfer von einem Gerät zu einem anderen, das heißt Modularität wird hier im Sinne eines Baukastensystems verstanden. Aufgrund seines modularen Aufbaus kann das System somit flexibel in andere Geräte integriert werden, ohne dass dazu größere technische Änderungen am Gerät vorgenommen werden müssen.

Weitere Vorteile des modularen Aufbaus sind darin zu sehen, dass eine eventuell vorhandene Elektronikeinheit, beispielsweise mit einem Bedien-Display ausgestattet, nicht im Bereich des heißen Öls angeordnet sein muss, sondern von dem Ölbecken in ausreichendem Maße beabstandet sein kann, sodass sie besser gekühlt werden kann und dass sie insbesondere flexibel an dem Ölbecken, je nach individuellen Erfordernissen des Benutzers, angeordnet werden kann.

Die erfindungsgemäße Ausgestaltung des Ölmessgeräts nach Anspruch 1 erlaubt weiter eine stationäre oder quasistationäre Überwachung der Qualität eines Ölbades. Unter Ölbad kann hier ein Behälter eines Geräts verstanden werden, der mit flüssigem Öl oder Fett, insbesondere aufgrund von Erhitzung flüssigem Öl oder Fett, befüllt ist. Der Begriff stationäre Messung kann hier so verstanden werden, dass das System vorzugsweise an einem ortsfesten Gerät betrieben wird, während der Begriff quasistationäre Messung so verstanden werden kann, dass das System vorzugsweise an einem zumindest übergangsweise ortsfesten Gerät betrieben wird.

Ein erfindungsgemäßes Messprinzip besteht darin, zur Überwachung des Öls einerseits die Temperatur des Öls und andererseits die Qualität des Öls zu messen, und zwar jeweils außerhalb des Ölbeckens. Zur Überwachung der Ölqualität kann das Öl somit zyklisch, also in durch Pausen unterbrochenen, zeitlichen Abständen, von dem Ölbad in die Messkammer gepumpt werden, um dort vermessen zu werden, wobei das Öl nach erfolgter Messung dem Ölbad wieder zugeführt werden kann. Diese Messmethode ist erfindungsgemäß bevorzugt. Falls erforderlich kann jedoch auch eine kontinuierliche Überwachung der Ölqualität durch ein erfindungsgemäßes Ölmessgerät oder Verfahren erreicht werden, bei welchem das Öl kontinuierlich durch die Messkammer gepumpt und dort vermessen wird.

Erfindungsgemäß kann die Aufgabe auch durch weitere vorteilhafte Ausführungen der Unteransprüche gelöst werden.

Bei einer vorteilhaften Ausgestaltung kann vorgesehen sein, dass das Ölmessgerät eine Elektronikeinheit aufweist. Diese Elektronikeinheit kann zur Ansteuerung der Pumpe und/oder zur Bestimmung der Ölqualität und/oder zur sonstigen Überwachung und Regelung von Funktionen des Ölmessgeräts und/oder zur Ansteuerung eines Displays mit oder ohne Eingabeeinheit eingesetzt werden. Somit kann eine Messung und/oder Überwachung der Ölqualität oder Öltemperatur durch das Elektronikgerät gesteuert, insbesondere automatisiert, ablaufen.

Die Elektronikeinheit befindet sich dabei vorzugsweise außerhalb des mit Öl befüllten Beckens. Diese Ausgestaltung hat den Vorteil, dass die Elektronikeinheit vor übermäßiger Überhitzung durch das heiße Öl geschützt werden kann und insbesondere auch besser gekühlt werden kann.

Eine weitere erfindungsgemäße Ausführungsform sieht vor, dass die Sonde eine Öl transportierende Schlauch- oder Rohrleitung als eine gemeinsame Zu-/Rückleitung oder doppelt ausgeführte Schlauch- oder Rohrleitungen, vorzugsweise als getrennte Zu-/Rückleitungen, aufweist. Die Sonde des Ölmessgeräts kann somit einen oder mehrere Sondenschläuche oder ein oder mehrere Sondenrohre aufweisen. Dabei kann das Öl mittels einer Zuleitung, vorzugsweise in Form einer Schlauch- oder Rohrleitung, der Messzelle zugeführt werden und mittels einer Rückleitung von der Messzelle wieder abgeführt werden. Alternativ kann die Zu- und Rückleitung auch durch eine einzige Schlauch- oder Rohrleitung ausgebildet sein, durch die der Messzelle im Wechsel entweder Öl zu- oder abgeführt werden kann. Somit kann insbesondere die Pumpe über die Rückleitung das Öl dem Ölbad des Geräts wieder zurückführen.

Die Probennahme kann somit mittels eines Kreislaufs geschehen, indem über eine Zuleitung Öl aus dem Becken entnommen, der Messkammer zugeführt und anschließend über eine Rückleitung dem Becken wieder zugeführt wird. Alternativ kann die Pumpe des Ölmessgeräts auch das Öl zuerst über eine Schlauch- oder Rohrleitung der Messkammer zuführen und anschließend, durch Umkehrung der Pumprichtung, über dieselbe Schlauch- oder Rohrleitung wieder an das Ölbecken zurückführen.

Die Sonde kann erfindungsgemäß somit flexibel oder starr ausgebildet sein. Die Sonde kann weiter zur Hin- und Rückleitung von Öl zwischen der Einlassöffnung und der Messkammer eingerichtet sein. Insbesondere kann die Sonde als Tauchsonde ausgebildet sein. Von Vorteil ist dabei, dass die Tiefe der Entnahmestelle von Öl in dem Becken durch die Eintauchtiefe der Sonde einstellbar ist.

Je nach Anwendung kann durch die Ausgestaltung des Ölmessgeräts mit einer starren Sonde deren Positionierbarkeit im Becken verbessert werden, während eine flexible Ausgestaltung der Sonde von Vorteil sein kann, wenn die Sonde im Verhältnis zu den proximalen Komponenten, beispielsweise der Pumpe, veränderlich positionierbar sein soll. Die Ausgestaltung als Tauchsonde hat den Vorteil, dass insbesondere eine schwimmende Lagerung der Sonde in dem mit Öl befüllten Becken möglich sein kann und/oder dass die Sonde vorzugsweise zur Entnahme von Öl aus einer definierten Tiefe des Beckens eingerichtet sein kann.

Um ein Verstopfen eines Sondenrohrs oder eines Sondenschlauchs, oder etwa eine Beeinträchtigung der Messkammer zu verhindern, kann erfindungsgemäß vorgesehen sein, dass die Sonde ein Filter, vorzugsweise ein am distalen Ende der Sonde angeordnetes Filter, aufweist, der das zu untersuchende Öl vor Eintritt in die Messkammer filtert.

Eine weitere erfindungsgemäße Ausgestaltung sieht vor, dass im Bereich des distalen Endes der Sonde ein distaler Temperatursensor angeordnet ist. Durch diesen Sensor kann die Temperatur des Öls direkt im Becken ermittelt werden, und somit ein für eine Messung der Ölqualität geeigneter Zeitpunkt, insbesondere automatisiert, gewählt werden. Dazu kann vorzugsweise eine Elektronikeinheit zum Auslesen des distalen Temperatursensors und/oder zur zyklischen und/oder kontinuierlichen Überwachung der Öltemperatur eingerichtet sein, insbesondere die weiter oben bereits erwähnte Elektronikeinheit. Somit kann beispielsweise bei nicht genügend hoher Temperatur das Ölmessgerät erst dann mit der Förderung von Öl in die Messkammer beginnen, wenn eine vorab eingestellte Mindesttemperatur erreicht ist.

Eine Weiterbildung dieser spezifischen Ausgestaltung sieht vor, dass ein distaler Temperatursensor als Thermoelement ausgebildet ist. Von Vorteil ist somit, dass im Handel erhältliche hochpräzise und kostengünstige Sensoren verwendet werden können.

Zusätzlich oder alternativ kann auch vorgesehen sein, dass ein distaler Temperatursensor benachbart zu einer Öl transportierenden Leitung angeordnet ist. Besonders vorteilhaft ist es dabei, wenn der distale Temperatursensor in unmittelbarer Nachbarschaft zu, insbesondere in wärmeleitendem Kontakt zu, einer Öl transportierenden Leitung und/oder im Bereich der Einlassöffnung (3) angeordnet ist. Durch diese Ausgestaltungen kann auf besonders einfache Weise die Öltemperatur im Becken mit hoher Genauigkeit erfasst werden, wobei der Temperatursensor dazu nicht notwendigerweise in direkten Kontakt mit dem Öl sein muss.

Eine weitere Ausbildung der Erfindung sieht vor, dass Mittel zur elektrischen Messung, insbesondere kapazitiven Messung, der Permittivität von in der Messkammer befindlichem Öl als Maß für die Ölqualität vorhanden sind und/oder dass die Elektronikeinheit zur Erfassung der gemessenen Ölqualität eingerichtet ist. Insbesondere kann erfindungsgemäß ein Koaxial-Sensor zur kapazitiven Vermessung der Messkammer vorgesehen sein. Wie bereits eingangs erwähnt, stellt die Messung der Permittivität von Öl ein gängiges und gut etabliertes Verfahren zur Bestimmung der Ölqualität dar. Somit ist auf einfache Weise eine hochpräzise Messung der Ölqualität möglich, wobei dazu nur sehr geringe Mengen an Öl aus dem Becken entnommen werden müssen.

Eine Weiterbildung dieser elektrischen Messung der Ölqualität sieht vor, dass das Ölmessgerät einen proximalen Temperatursensor zur Erfassung der Öltemperatur aufweist. Dieser proximale Temperatursensor kann beispielsweise in unmittelbarer Nachbarschaft der Messkammer, vorzugsweise in wärmeleitendem Kontakt zur Messkammer, und/oder in der Messkammer angeordnet sein. Hierbei kann vorzugsweise die Elektronikeinheit zum Auslesen des proximalen Temperatursensors eingerichtet sein. Durch das Bereitstellen der in oder in Nähe der Messzelle herrschenden Öltemperatur kann somit eine noch präzisere Messung der Permittivität und damit der Ölqualität erreicht werden, da die nicht zu vernachlässigende Temperaturabhängigkeit der elektrischen Permittivität des Öls in der Messkammer bei der Interpretation der Messdaten berücksichtigt werden kann.

Eine besonders gut zu handhabende Ausgestaltung der Erfindung sieht vor, dass die proximalen Komponenten, also die Messkammer, die Pumpe, die Mittel zur Bestimmung der Qualität des Öls und eine eventuell vorhandene Elektronikeinheit, sowie vorzugsweise ein proximaler Temperatursensor, in oder an einem gemeinsamen Gehäuse zusammengefasst sind. Somit kann das Ölmessgerät insbesondere in Form eines portablen Handgeräts mit an dem Gehäuse anschließbarer Sonde vorliegen. Die Zusammenfassung von Komponenten in einem Gehäuse ermöglicht dabei einen effizienten Schutz vor Beschädigungen, insbesondere durch Kontakt mit heißem Öl sowie eine kompakte Bauweise des Ölmessgeräts insgesamt.

Auf dieser Ausgestaltung aufbauend kann auch vorgesehen sein, dass eine Position der Sonde relativ zur Position eines die proximalen Komponenten aufnehmenden Gehäuses veränderbar ist. Damit können die Gebrauchseigenschaften der Sonde wesentlich verbessert werden, da die Sonde unabhängig von dem Gehäuse positioniert werden kann, was für die Verwendung des Ölmessgeräts in verschieden ausgelegten Becken von Vorteil sein kann. Insbesondere kann somit die Einlassöffnung der Sonde relativ zur Pumpe flexibel positioniert werden, was die Bedienbarkeit der Sonde wesentlich verbessern kann.

Eine besonders einfache und daher attraktive Ausgestaltung des erfindungsgemäßen Ölmessgeräts ergibt sich, wenn die Pumpe in Form einer Peristaltikpumpe ausgebildet ist und/oder wenn die Pumpe zwischen Messkammer und Einlassöffnung angeordnet ist. Dabei kann insbesondere auch vorgesehen sein, dass die Pumprichtung der Pumpe umkehrbar ist. Durch derartige Ausgestaltungen kann beispielsweise eine einzige Leitung in Form eines Rohres oder eines Schlauchs als Zu- und Rückleitung für Öl genutzt werden, was hilfreich sein kann, um den konstruktiven Aufwand für die Sonde gering zu halten.

Weitere Vorteile in der Benutzung des Ölmessgeräts ergeben sich, wenn die Elektronikeinheit einen Datenspeicher zur Speicherung erfasster Messwerte und/oder eine Schnittstelle, vorzugsweise eine drahtlose Schnittstelle, aufweist. Besonders von Vorteil ist es dabei, wenn die Schnittstelle zur Ausgabe erfasster und/oder gespeicherter Messdaten eingerichtet ist. Die Schnittstelle kann also mit gängigen Mitteln der Signalübertragung ausgebildet sein, etwa mit Steckern für Kabel oder mit einer Funkeinheit, zum Beispiel in Form eines RF-Transmitters. Insbesondere kann die Schnittstelle auch ein bedienbares Anzeigen-Display aufweisen.

Mit einer derart ausgestalteten Elektronikeinheit können somit Messwerte, die über längere Zeiträume erfasst wurden, in dem Gerät gespeichert und zu geeigneten Zeitpunkten über die Schnittstelle von einem Benutzer abgerufen werden, also an diesen ausgegeben werden, beispielsweise um die Daten weiterverarbeiten zu können oder lediglich zum Zwecke einer Dokumentation von Messdaten. Eine drahtlose Schnittstelle bietet dabei den Vorteil, dass der Benutzer nicht in die unmittelbare Nähe des Ölmessgeräts gelangen muss, um derartige Daten von dem Ölmessgerät abzurufen.

Die Effizienz und Genauigkeit der Messung der Ölqualität in der Messkammer kann erfindungsgemäß weiter verbessert werden, indem eine Vorrichtung zur Detektion und/oder Anzeige des Füllstands des Öls in der Messkammer vorhanden ist, wobei vorzugsweise die Elektronikeinheit Bestandteil dieser Vorrichtung ist. Somit kann beispielsweise entweder rein optisch von außen sichtbar, oder durch elektronische Sensorik der Füllstand des Öls in der Messkammer bis zu einem Mindestfüllstand erfassbar sein, ab dem eine Messung der Ölqualität als aussagekräftig erachtet werden kann. Weiter können durch die Füllstandsanzeige beispielsweise auch Luftblasen in der Messkammer, welche eine Messung verfälschen könnten, detektierbar sein.

Aufgrund der einfachen Umsetzung sieht eine besonders bevorzugte Ausführungsform dazu vor, dass das Ölmessgerät Mittel zur Detektion des Füllstands des Öls in der Messkammer durch Überwachung des kapazitiven Verhaltens der Messkammer aufweist. Zusätzlich oder alternativ kann auch vorgesehen sein, dass die Elektronikeinheit zum Starten einer, vorzugsweise kapazitiven, Messung der Ölqualität nach Detektion eines Mindestfüllstands des Öls in der Messkammer eingerichtet ist. Somit kann auf besonders einfache Weise, vorzugsweise mit eventuell bereits für die Messung der Ölqualität vorhandenen Mitteln, der Füllstand des Öls in der Messkammer detektiert werden und somit ein geeigneter Zeitpunkt für das Starten einer Messung durch das Ölmessgerät bestimmbar sein.

Eine weitere vorteilhafte Weiterbildung der Erfindung sieht vor, dass das Ölmessgerät ein Umschaltventil aufweist, an welchem mindestens eine weitere erfindungsgemäße Sonde anordenbar, insbesondere reversibel anordenbar, ist. Dabei kann mindestens eine Pumpe und mindestens eine Messkammer mit dem Umschaltventil in Verbindung stehen. Somit kann insbesondere Öl aus mehreren Sonden von einer gemeinsamen Pumpe in eine gemeinsame Messkammer förderbar sein oder gefördert werden. Weiter kann auch vorgesehen sein, dass das Umschaltventil Mittel zur Verfügung stellt, um in den Sonden integrierte distale Temperatursensoren über eine Elektronikeinheit des Ölmessgeräts auszulesen. Die anschließbaren Sonden können somit auch als Temperaturfühler ohne Öl fördernde Eigenschaften ausgelegt sein. Durch diese Ausgestaltungen kann somit der flexible Einsatz eines erfindungsgemäßen Ölmessgeräts weiter verbessert werden.

Weitere Verbesserungen der Gebrauchseigenschaften des Ölmessgeräts können erfindungsgemäß dadurch erreicht werden, dass die Sonde, vorzugsweise das Ölmessgerät, an einem ein zu untersuchendes Öl aufnehmendes Becken in der Position veränderlich, insbesondere abnehmbar, befestigbar ist. Insbesondere kann somit vorgesehen sein, dass die Sonde, vorzugsweise das Ölmessgerät, an mehreren Becken modular verwendet werden kann, wodurch sich das mögliche Einsatzgebiet der Sonde erweitert.

Zur Lösung der genannten Aufgabe sind erfindungsgemäß die Merkmale des unabhängigen Verfahrensanspruchs vorgesehen. Insbesondere wird somit erfindungsgemäß zur Lösung der Aufgabe bei einem Verfahren zur Messung der Qualität eines in einem Becken befindlichen Öls wie eingangs beschrieben vorgeschlagen, dass ein Teil des Öls mittels einer Sonde, vorzugsweise mittels einer Sonde eines Ölmessgeräts nach einem der auf ein Ölmessgerät gerichteten Ansprüche, aus dem Becken entnommen, vermessen und anschließend über dieselbe Sonde an das Becken zurückgegeben wird, insbesondere wobei das Öl mittels einer Pumpe gefördert wird. Dieses Verfahren bietet den entscheidenden Vorteil, dass für eine Qualitätsprüfung nur sehr geringe Mengen des Öls aus dem Becken entnommen werden müssen, sodass insbesondere Totvolumina in Leitungen, wie sie bei herkömmlichen Systemen auftreten, vermieden werden können. Daneben sind allenfalls nur marginale konstruktive Veränderungen an dem Becken notwendig, um ein solches Verfahren einsetzen zu können. Durch das erfindungsgemäße Verfahren lässt sich somit mittels einfacher technischer Mittel ein in einem Becken befindliches Öl, vorzugsweise automatisiert, überwachen. Dadurch können während des Betriebs vorgeschriebene Qualitätsstandards in Bezug auf das Öl eingehalten werden.

Ein weiteres auf dem gerade diskutierten Verfahren aufbauendes, erfindungsgemäßes Verfahren sieht vor, dass zur Bestimmung der Ölqualität eines in einem Becken befindlichen Öls dieses Öl wie in Verfahrensanspruch 17 beschrieben aus dem Becken entnommen wird, dann in einer an die Sonde angeschlossenen Messkammer vermessen wird und anschließend wieder über dieselbe Sonde an das Becken zurückgegeben wird. Dabei ist es vorteilhaft wenn, vorzugsweise zeitlich vor oder während der Ölqualitätsmessung, die Temperatur des Öls am distalen Ende der Sonde und/oder am proximalen Ende der Sonde, in letzterem Fall vorzugsweise in der Messkammer, erfasst wird. Dieses bevorzugte Verfahren stellt somit sicher, dass eine hohe Genauigkeit der Messung erreichbar ist, durch Verwendung einer für die Messung spezifisch ausgelegten Messzelle und/oder durch Berücksichtigung von Temperatureffekten in dem Ölbecken und/oder am Ort der Messung.

Eine Temperaturüberwachung kann erfindungsgemäß auch der Überwachung von Temperaturzyklen dienen, wobei dieses Verfahren insbesondere zur Überwachung von Frittierzyklen bei Fritteusen eingesetzt werden kann: Beim Einlegen von zu frittierenden Lebensmitteln, insbesondere bei Gefriergut, in die Fritteuse kommt es typischerweise zu einem kurzzeitigen Absenken der Öltemperatur, die danach wieder auf die Betriebstemperatur steigt, was von einem Verdunsten von Wasser aus dem Öl begleitet wird. Der Erfindung liegt der Ansatz zu Grunde, dieses Absenken der Temperatur mittels des Temperatursensors aufzunehmen und abzuspeichern, sodass aus den ermittelten Daten Rückschlüsse auf die Dauer und Anzahl der Frittierzyklen gezogen werden können.

Durch eine erfindungsgemäße, vorzugsweise zeitlich zyklische oder kontinuierliche, Messung der Ölqualität sowie der Temperatur des Öls kann somit beispielhaft für eine Fritteuse eine Überwachung insbesondere folgender Parameter erreicht werden: zeitliche Auslastung der Fritteuse, Anzahl und Dauer von Frittierzyklen, Betriebsdauer der Fritteuse, sowie Temperaturschwankungen des Ölbades während des Betriebs der Fritteuse.

Zur Lösung der genannten Aufgabe sind daher alternativ die Merkmale eines zweiten, nebengeordneten Verfahrensanspruchs 19 vorgesehen, der möglicherweise eigenständigen erfinderischen Charakter hat. Insbesondere wird somit erfindungsgemäß zur Lösung der Aufgabe bei einem Verfahren zur Messung der Anzahl und/oder Dauer von Erhitzungszyklen eines in einem Becken befindlichen Öls wie eingangs beschrieben vorgeschlagen, dass mittels einer Sonde, vorzugsweise mittels einer Sonde eines Ölmessgeräts nach einem der auf ein solches gerichteten Ansprüche, der zeitliche Verlauf der Temperatur des Öls erfasst wird und daraus die Anzahl und Dauer der Erhitzungszyklen berechnet wird. Dabei kann die Erfassung der Temperatur vorzugsweise zeitlich zyklisch und/oder zeitlich kontinuierlich erfolgen. Unter Kontinuierlicher Erfassung kann hier insbesondere verstanden werden, dass Messwerte fortlaufend, während des Betriebs des Ölbeckens aufgezeichnet werden.

Weiter kann zur Bestimmung der Anzahl und Dauer der Erhitzungszyklen auch vorgesehen sein, dass anhand von unter- oder überschrittenen Temperaturschwellwerten und/oder anhand von gemessenen zeitlichen Temperaturänderungsraten, Temperaturabfälle oder Temperaturanstiege detektiert werden, aus welchen die Anzahl und/oder Dauer der Erhitzungszyklen abgeleitet werden kann. Insbesondere kann hierbei auch vorgesehen sein, dass die Anzahl und Dauer der Erhitzungszyklen zum Zwecke der Weiterverarbeitung, vorzugsweise in dem zur Erfassung genutzten Gerät abspeicherbar sind oder abgespeichert werden und von diesem ausgebbar sind oder von diesem ausgegeben werden.

Besonders günstig ist es hierbei, wenn in den erfindungsgemäßen Verfahren jeweils ein erfindungsgemäßes Ölmessgerät mit erfindungsgemäßer Sonde, insbesondere wie zuvor beschrieben oder nach einem der auf ein Ölmessgerät gerichteten Schutzansprüche, verwendet wird.

Die Erfindung wird nun anhand von Ausführungsbeispielen näher beschrieben, ist aber nicht auf diese Ausführungsbeispiele beschränkt. Weiterbildungen gemäß der Erfindung ergeben sich aus der Kombination der Merkmale wenigstens eines Anspruchs mit den Merkmalen einzelner oder mehrerer Unteransprüche untereinander und/oder mit der nachfolgenden Beschreibung eines bevorzugten Ausführungsbeispiels, insbesondere in Verbindung mit der allgemeinen Beschreibung, den Ansprüchen sowie den Zeichnungen.

Es zeigt:
- Fig. 1: den als Ausgangspunkt der Erfindung relevanten Stand der Technik, dargestellt als Querschnittsansicht eines mit Öl befüllten Beckens 19, an welchem ein Öl zirkulierender Kreislauf samt Pumpe 5 angeschlossen ist, wobei in dem Kreislauf Sensorik zur Messung der Ölqualität an unterschiedlichen Stellen fest verbaut ist,
- Fig. 2: ein beispielhaft in Querschnittsansicht dargestelltes mit Öl befülltes Becken 19, wobei eine erfindungsgemäße Sonde 2 in das Öl Becken eintaucht, um Öl aus dem Becken zu entnehmen und zu vermessen,
- Fig. 3: eine stark schematisierte Ansicht eines Gehäuses 17 eines erfindungsgemäßen Ölmessgerät 1 mit darin enthaltenen, für die Erfindung typischen Komponenten.
- Fig.4: eine schematisierte Darstellung eines erfindungsgemäßen Ölmessgeräts 1 mit zwei erfindungsgemäßen Sonden 2, die über ein Umschaltventil 16 anschließbar sind.

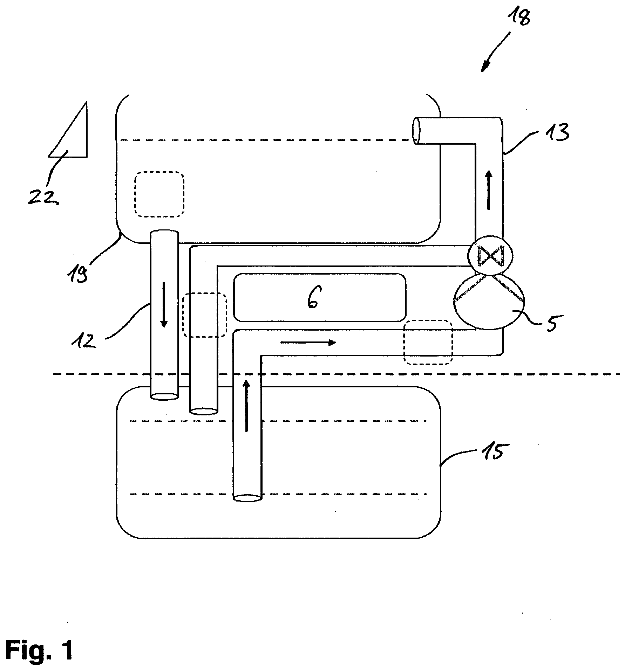

Fig. 1 zeigt ein für den Stand der Technik typisches Gerät 18 mit einem Display mit Eingabeeinheit 22 und einem mit Öl befüllten Becken 19, bei welchem über Öl ab- und zuführende Leitungen 12, 13 sowie eine Pumpe 5 ein Kreislauf gebildet ist, der Öl aus dem Becken 19, durch ein Filter 15 und zurück in das Becken 19 zirkuliert. Wie durch die gestrichelten Boxen angedeutet, sind zur Überwachung des Öls Sensoren typischerweise an einzelnen oder mehreren Stellen im Kreislauf oder im Ölbecken 19 fest verbaut. Für bestehende Ölbecken mit einer Öl zirkulierenden Pumpe 5 bedeutet dies, dass eine derartige Sensorik nur über größere Umbauten an dem Ölbecken 19 sowie dem zugehörigen Leitungssystem nachrüstbar ist.

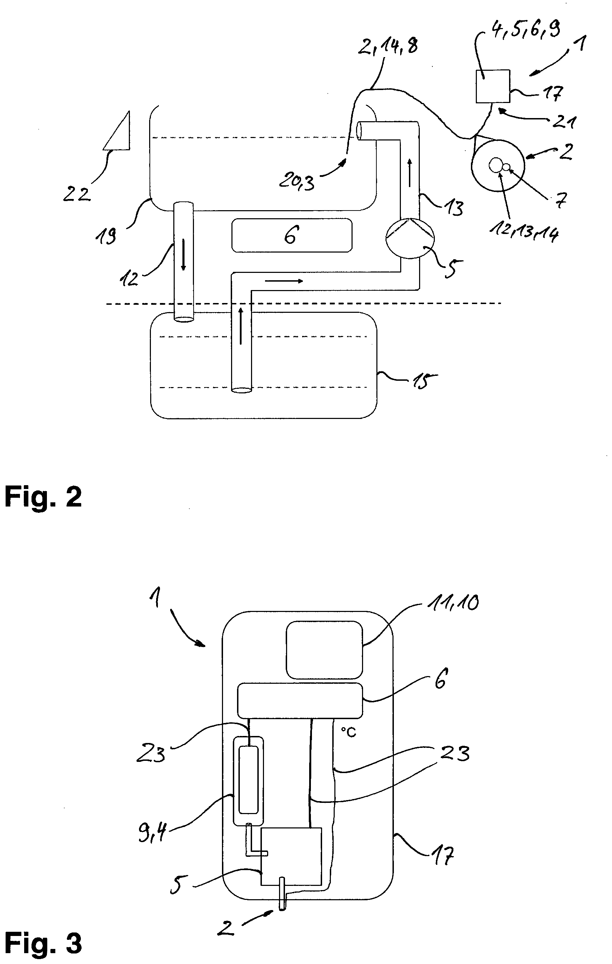

Fig. 2 veranschaulicht, wie ein erfindungsgemäßes Ölmessgerät 1 zur Überwachung eines bestehenden Ölbeckens 19 eingesetzt werden kann. Dazu muss erfindungsgemäß lediglich die Sonde 2 in das Öl eingebracht werden, sodass über eine Einlassöffnung 3 am distalen Ende 20 der Sonde 2 Öl aus dem Becken entnommen und einer an die Sonde angeschlossenen Messkammer 4 des Ölmessgeräts mittels einer im Ölmessgerät integrierten Pumpe 5 zugeführt werden kann. Dies kann im einfachsten Fall durch eine in der Sonde integrierte oder durch sie ausgebildete Zu-12 und Rückleitung 13 in Form eines Schlauchs 14 geschehen. Alternativ können auch doppelt ausgeführte Schlauch- oder Rohrleitungen 14, vorzugsweise als getrennte Zu-/ Rückleitungen 12, 13, in der oder durch die Sonde ausgebildet sein. Insbesondere kann die Pumpe derart betrieben werden, dass diese in der Messkammer 4 untersuchtes Öl über eine Schlauch- oder Rohrleitung 14 an das Ölbecken 19 zurückfördert. Um die Qualität des Öls zu vermessen sind erfindungsgemäß Messmittel vorgesehen, die in Fig. 2 nicht näher dargestellt sind, die sich jedoch insbesondere am proximalen Ende der Sonde in oder an der Messkammer 4 befinden können. Das Ölmessgerät kann weiter eine Elektronikeinheit 6 sowie einen proximalen Temperatursensor 9 umfassen, die vorzugsweise in einem am proximalen Ende 21 der Sonde 2 angeordneten Gehäuse 17 untergebracht sind. Die Elektronikeinheit kann dabei vorzugsweise zur Ansteuerung der Pumpe und oder zu Bestimmung der Ölqualität eingerichtet sein. Wie das Detail in Fig.2 zeigt, kann erfindungsgemäß ein distaler Temperatursensor 7, insbesondere benachbart zu einer Öl transportierenden Leitung 14, in oder an der Sonde 2 angeordnet sein, wobei eine Anordnung im Bereich des distalen Endes 20 der Sonde 2 bevorzugt ist. Dieser Temperatursensor kann insbesondere als Thermoelement ausgebildet sein. Durch die Ausgestaltung des Ölmessgeräts mit einem kompakten und leichten Gehäuse 17 und einer davon unabhängig bewegbaren Sonde 2 ist es insbesondere, wie in Fig. 2 angedeutet, möglich, eine Position der Sonde 2 relativ zur Position des Gehäuses 17 zu verändern, und auch, das Ölmessgerät 1 insgesamt in der Position veränderlich, insbesondere abnehmbar, an dem Ölbecken 19 zu befestigen. Dadurch wird die Handhabung des Ölmessgeräts 1 wesentlich verbessert.

Fig. 3 zeigt eine schematisierte Ansicht eines Gehäuses 17 eines erfindungsgemäßen Ölmessgeräts 1: Wie durch die Bezeichner angedeutet, kann ein solches Gehäuse und damit das Ölmessgerät eine Messzelle 4, eine Pumpe 5, eine Schnittstelle 1, sowie eine Elektronikeinheit 6 umfassen, wobei die Elektronikeinheit 6 über Steuer- und Versorgungsleitungen 23 mit den anderen Komponenten wie abgebildet verbunden sein kann. Insbesondere kann somit die Elektronikeinheit zum Auslesen und/oder Steuern der Messzelle 4, und/oder zum Auslesen und/oder Steuern von distalen und/oder proximalen Temperatursensoren 6,7 und/oder einer Schnittstelle, beispielsweise einer drahtlosen RF-Schnittstelle, und/oder zur Erfassung einer gemessenen Ölqualität eingerichtet sein. Somit kann das Ölmessgerät 1 auch Mittel zur elektrischen Messung, insbesondere kapazitiven Messung, der Permittivität von in der Messkammer 4 befindlichem Öl aufweisen. Um dabei eine noch höhere Messgenauigkeit zu erreichen ist es bevorzugt, wenn ein zusätzlicher proximaler Temperatursensor 9 vorgesehen ist, welcher, wie in Fig.2 ersichtlich, insbesondere in unmittelbarer Nachbarschaft der Messkammer und/oder in der Messkammer 4 eine Temperatur erfasst. Eine sehr kompakte Bauform des Gehäuses 17 insgesamt lässt sich erreichen, wenn die Pumpe 5 als Peristaltikpumpe ausgebildet ist. Weitere Verbesserungen des Ölmessgeräts 1 ergeben sich, wenn, wie in Fig.3 dargestellt, die Elektronikeinheit 6 einen Datenspeicher 10 zur Speicherung erfasster Messwerte und/oder eine Schnittstelle 11, vorzugsweise eine drahtlose Schnittstelle 11, aufweist. Damit lassen sich auf sehr einfache Weise Messdaten zwischenspeichern und zur weiteren Verarbeitung ausgeben. Das Ölmessgerät 1 kann auch im Bereich der Messkammer 4 eine in Fig. 3 nicht näher dargestellte Vorrichtung zur Detektion und/oder Anzeige des Füllstands des Öls in der Messkammer 4 aufweisen, wobei vorzugsweise die Elektronikeinheit Bestandteil dieser Vorrichtung ist. Diese Vorrichtung kann somit beispielsweise in Form eines einfachen Sichtfensters an der mit Öl befüllbaren Messkammer, als eine Signalleuchte, oder etwa als Anzeige in einem Display 22 des Ölmessgeräts 1 vorliegen. Es kann darüber hinaus auch vorgesehen sein, dass das Ölmessgerät 1 Mittel zur Detektion des Füllstands des Öls in der Messkammer 4 mittels einer kapazitiven Messung aufweist, wobei begleitend oder alternativ auch die Elektronikeinheit 6 zum Starten einer, vorzugsweise kapazitiven, Messung der Ölqualität nach Detektion eines Mindestfüllstands des Öls in der Messkammer 4 eingerichtet sein kann. Wie in Fig.3 dargestellt, wird diese Funktionalität durch die zwischen den einzelnen Komponenten des Gehäuses 17 verlaufenden Steuer- und Versorgungsleitungen 23 ermöglicht. Die Sonde 2 kann an dem Gehäuse 17 reversibel anbringbar oder mit diesem fest verbaut sein. Wie durch Fig. 3 angedeutet, ist die Pumpe 5 vorzugsweise zwischen der Messzelle 4 und der Sonde 2 angeordnet.

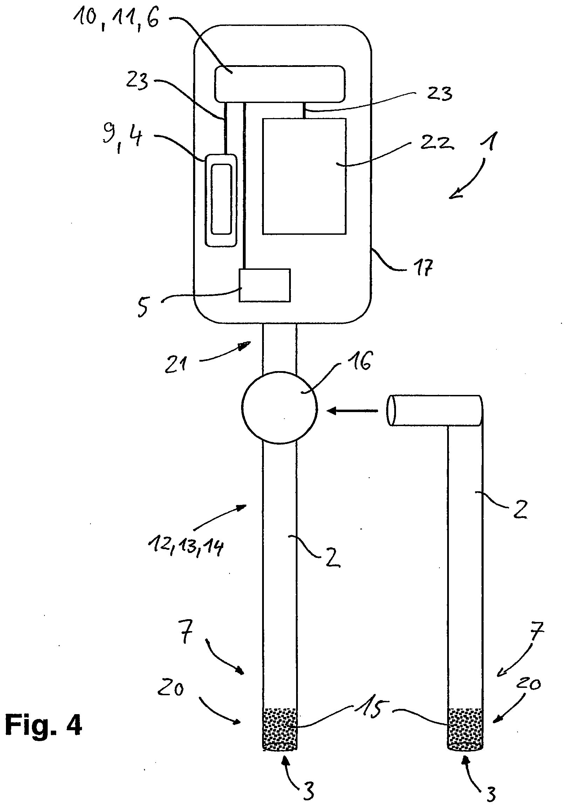

Schließlich zeigt Fig. 4, wie mit Hilfe eines Umschaltventils 16 weitere erfindungsgemäße Sonden 2 an dem Ölmessgerät 1 anschließbar beziehungsweise anordenbar sind. Die Sonden können jeweils mit distalen Temperatursensoren 7 und Filtern 15 ausgestattet sein und besitzen in Form von Schlauch- oder Rohrleitungen 14 vorliegende Zu- und Rückleitungen 12, 13 beziehungsweise bilden derartige Leitungen aus. Das Filter, welches das zu untersuchende Öl vor Eintritt in die Messkammer filtert, kann auch innerhalb des Gehäuses 17 angeordnet werden, bevorzugt jedoch, wie in Fig.4 abgebildet, am distalen Ende 20 der Sonde 2. In der in Fig. 4 gezeigten Ausführungsform weist das Gehäuse 17 am proximalen Ende 21 der Sonde 2 beispielhaft eine Elektronikeinheit 6 mit Datenspeicher 10 und Schnittstelle 11, eine Messzelle 4 mit integriertem proximalen Temperatursensor 9, eine Pumpe 5 sowie ein Display 22 mit Eingabeeinheit auf.

Zur Überwachung eines in einem Becken 19 befindlichen Öls wird vorgeschlagen, ein Ölmessgerät 1 mit einer starren oder flexiblen Sonde 2 auszustatten, sodass mit Hilfe einer in dem Ölmessgerät 1 integrierten Pumpe 5 Öl aus dem Becken 19 über eine Einlassöffnung 3 am proximalen Ende der Sonde 2 in eine an die Sonde angeschlossene Messkammer 4 des Ölmessgeräts 1 förderbar ist. Dabei weist das Ölmessgerät 1 ein Messmittel zur Bestimmung der Qualität von in der Messkammer 4 befindlichem Öl auf und die Pumpe 5, in Verbindung mit der Sonde 2, ist so eingerichtet, dass Öl von der Messkammer 4 an die Einlassöffnung 3 und damit an das Becken 19 rückführbar ist.

### Bezugszeichenliste

- 1: Ölmessgerät
- 2: Sonde
- 3: Einlassöffnung
- 4: Messkammer
- 5: Pumpe
- 6: Elektronikeinheit
- 7: Distaler Temperatursensor
- 8: Leitung
- 9: Proximaler Temperatursensor
- 10: Datenspeicher
- 11: Schnittstelle
- 12: Zuleitung
- 13: Rückleitung
- 14: Schlauch- oder Rohrleitung
- 15: Filter
- 16: Umschaltventil
- 17: Gehäuse
- 18: Gerät
- 19: Ölbecken
- 20: Distales Ende der Sonde
- 21: Proximales Ende der Sonde
- 22: Display mit Eingabeeinheit
- 23: Steuer- und Versorgungsleitungen

## Patentansprüche

1. Ölmessgerät (1) mit einer Sonde (2), die an ihrem distalen Ende (20) eine Einlassöffnung (3) aufweist, wobei an einem proximalen Ende (21) der Sonde (2) eine mit Öl befüllbare und an die Sonde angeschlossene Messkammer (4) und eine Pumpe (5) zur Förderung von Öl von der Einlassöffnung (3) zur Messkammer (4) angeordnet sind, wobei das Ölmessgerät (1) ein Messmittel zur Bestimmung der Qualität des Öls aufweist und wobei die Pumpe (5) zur Rückführung von in der Messkammer (4) untersuchtem Öl zur Einlassöffnung (3) eingerichtet ist.

2. Ölmessgerät (1) nach Anspruch 1, **dadurch gekennzeichnet, dass** das Ölmessgerät (1) eine Elektronikeinheit (6), vorzugsweise zur Ansteuerung der Pumpe (5) und/oder zur Bestimmung der Ölqualität, aufweist.

3. Ölmessgerät (1) nach einem der vorhergehenden Ansprüche, **dadurch gekennzeichnet, dass** die Sonde (2) eine Öl transportierende Schlauch- oder Rohrleitung (14) als eine gemeinsame Zu-/Rückleitung (12,13) oder doppelt ausgeführte Schlauch- oder Rohrleitungen (14), vorzugsweise als getrennte Zu-/Rückleitungen (12,13), aufweist und/oder ausbildet.

4. Ölmessgerät (1) nach einem der vorhergehenden Ansprüche, **dadurch gekennzeichnet, dass** die Sonde (2) ein Filter (15), vorzugsweise ein am distalen Ende (20) der Sonde (2) angeordnetes Filter (15), aufweist, der das zu untersuchende Öl vor Eintritt in die Messkammer (4) filtert.

5. Ölmessgerät (1) nach einem der vorhergehenden Ansprüche, **dadurch gekennzeichnet, dass** die Sonde (2), bevorzugt im Bereich ihres distalen Endes (20), einen distalen Temperatursensor (7) aufweist.

6. Ölmessgerät (1) nach Anspruch 5, **dadurch gekennzeichnet, dass** der distale Temperatursensor (7) als Thermoelement ausgebildet ist und/oder dass der distale Temperatursensor (7) benachbart zu einer Öl transportierenden Leitung (8) angeordnet ist.

7. Ölmessgerät (1) nach einem der vorhergehenden Ansprüche, **dadurch gekennzeichnet, dass** das Ölmessgerät (1) Mittel zur elektrischen Messung, insbesondere kapazitiven Messung, der Permittivität von in der Messkammer (4) befindlichem Öl aufweist und/oder dass die Elektronikeinheit (6) zur Erfassung einer gemessenen Ölqualität eingerichtet ist.

8. Ölmessgerät (1) nach einem der vorhergehenden Ansprüche, **dadurch gekennzeichnet, dass** das Ölmessgerät einen proximalen Temperatursensor (9) zur Erfassung der Öltemperatur aufweist, vorzugsweise wobei der proximale Temperatursensor (9) in unmittelbarer Nachbarschaft der Messkammer (4) und/oder in der Messkammer (4) angeordnet ist.

9. Ölmessgerät (1) nach einem der vorhergehenden Ansprüche, **dadurch gekennzeichnet, dass** die proximalen Komponenten, also die Messkammer (4), die Pumpe (5), die Messmittel zur Bestimmung der Qualität des Öls und die Elektronikeinheit (6) sowie vorzugsweise der proximale Temperatursensor (9), in oder an einem gemeinsamen Gehäuse (17) zusammengefasst sind.

10. Ölmessgerät (1) nach Anspruch 9, **dadurch gekennzeichnet, dass** eine Position der Sonde (2) relativ zur Position eines die proximalen Komponenten des Ölmessgeräts (1) aufnehmenden Gehäuses (17) veränderbar ist.

11. Ölmessgerät (1) nach einem der vorhergehenden Ansprüche, **dadurch gekennzeichnet, dass** die Pumpe (5) in Form einer Peristaltikpumpe ausgebildet ist.

12. Ölmessgerät (1) nach einem der vorhergehenden Ansprüche, **dadurch gekennzeichnet, dass** die Elektronikeinheit (6) einen Datenspeicher (10) zur Speicherung erfasster Messwerte und/oder eine Schnittstelle (11), vorzugsweise eine drahtlose Schnittstelle (11), aufweist.

13. Ölmessgerät (1) nach einem der vorhergehenden Ansprüche, **dadurch gekennzeichnet, dass** das Ölmessgerät (1) eine Vorrichtung zur Detektion und/oder Anzeige des Füllstands des Öls in der Messkammer (4) aufweist, wobei vorzugsweise die Elektronikeinheit (6) Bestandteil dieser Vorrichtung ist.

14. Ölmessgerät (1) nach einem der vorhergehenden Ansprüche, **dadurch gekennzeichnet, dass** das Ölmessgerät Mittel zur Detektion des Füllstands des Öls in der Messkammer (4) mittels einer kapazitiven Messung aufweist und/oder dass die Elektronikeinheit (6) zum Starten einer, vorzugsweise kapazitiven, Messung der Ölqualität nach Detektion eines Mindestfüllstands des Öls in der Messkammer (4) eingerichtet ist.

15. Ölmessgerät (1) nach einem der vorhergehenden Ansprüche, **dadurch gekennzeichnet, dass** an einem Umschaltventil (16) mindestens eine weitere erfindungsgemäße Sonde (2) anordenbar ist, sodass Öl aus mehreren Sonden (2) von einer Pumpe (5) in eine Messkammer (5) gefördert werden kann.

16. Ölmessgerät (1) nach einem der vorhergehenden Ansprüche, **dadurch gekennzeichnet, dass** die Sonde (2), vorzugsweise das Ölmessgerät (1), an einem ein zu untersuchendes Öl aufnehmendes Becken (19) in der Position veränderlich, insbesondere abnehmbar, befestigbar ist.

17. Verfahren zur Messung der Qualität eines in einem Becken (19) befindlichen Öls, **dadurch gekennzeichnet, dass** ein Teil des Öls mittels einer Sonde (2) aus dem Becken (19) entnommen, vermessen und anschließend über dieselbe Sonde an das Becken (19) zurückgegeben wird, insbesondere wobei das Öl mittels einer Pumpe (5) gefördert wird.

18. Verfahren zur Vermessung eines in einem Becken (19) befindlichen Öls nach Anspruch 17, **dadurch gekennzeichnet, dass** die Ölqualität in einer an die Sonde angeschlossenen Messkammer (4) vermessen wird und/oder dass die Temperatur des Öls am distalen Ende (20) der Sonde (2) und/oder am proximalen Ende (21) der Sonde (2) erfasst wird.

19. Verfahren zur Messung der Anzahl und/oder Dauer von Erhitzungszyklen eines in einem Becken (19) befindlichen Öls, **dadurch gekennzeichnet, dass** mittels einer Sonde (2), vorzugsweise einer Tauchsonde, der zeitliche Verlauf der Temperatur des Öls erfasst wird und daraus die Anzahl und Dauer der Erhitzungszyklen berechnet wird.
